# EUROPÄISCHE PATENTSCHRIFT

(11) **EP 3 162 404 B1**
(45) Veröffentlichungstag und Bekanntmachung des Hinweises auf die Patenterteilung: **14.02.2018**
(21) Anmeldenummer: 15192001.4
(22) Anmeldetag: 29.10.2015
(51) Int. Cl.: A61C 3/025, A61M 13/00, A61M 15/00, B05B 11/06

(54) **DENTALMEDIZINISCHE VORRICHTUNG ZUR ABGABE EINES PULVERFÖRMIGEN MEDIUMS**
DENTAL DEVICE FOR DELIVERING A POWDERY MEDIUM
DISPOSITIF DENTAIRE DESTINE A LA DISTRIBUTION D'UN MILIEU PULVERULENT

(43) Veröffentlichungstag der Anmeldung: 03.05.2017
(73) Patentinhaber: W & H Dentalwerk Bürmoos GmbH, 5111 Bürmoos (AT)
(72) Erfinder: EDER, Karlheinz, 5152 Michaelbeuern (AT); PERWEIN, Wolfgang, 5112 Lamprechtshausen (AT); SPITZAUER, Josef, 5110 Loipferding (AT); HÖRMANN, Udo, 5411 Oberalm (AT)
(74) Vertreter: Benda, Ralf

(56) Entgegenhaltungen:
- WO-A1-02/056948
- DE-T2- 60 308 914
- US-A- 2 950 564
- US-A1- 2005 267 628
- US-A1- 2007 175 476
- US-A1- 2014 014 104

## Beschreibung

Die vorliegende Erfindung betrifft eine dentalmedizinische Vorrichtung zur Abgabe eines pulverförmigen, durch ein Druckgas fluidisierten Mediums auf menschliches oder tierisches Gewebe oder auf ein Gewebe-Ersatzmaterial.

Eine derartige Vorrichtung ist aus der Patentanmeldung WO 2007/014246 A2 bekannt. Die Vorrichtung zur Abgabe eines pulverförmigen Mediums ist zur Versorgung mit Druckgas und Wasser an eine Dentaleinheit anschließbar und umfasst einen Behälter zum Aufbewahren des pulverförmigen Mediums und eine Abgabeeinheit in Form eines Handstücks mit Düse über welche das pulverförmige Medium mit dem Druckgas auf eine Behandlungsstelle abgebbar ist. Zur Förderung des pulverförmigen Mediums aus dem Behälter zu der Abgabeeinheit wird dem Behälter und dem darin befindliche Pulver Druckgas zugeführt.

In der Praxis ist der Betrieb einer derartigen Vorrichtung mit konstanten Betriebsparametern schwierig. So ist häufig zu beobachten, dass die von dem Druckgas aus dem Behälter zu der Abgabeeinheit geförderte Menge des pulverförmigen Mediums stark variiert, zum Beispiel in Abhängigkeit des Füllstands des pulverförmigen Mediums in dem Behälter oder in Abhängigkeit der Art des pulverförmigen Mediums, z.B. dessen Korngröße. Da das in dem Behälter aufbewahrte pulverförmige Medium sehr fein ist, d.h. geringe Korngrößen aufweist, und oftmals hygroskopisch ist, kommt es zusätzlich häufig vor, dass das pulverförmige Medium in dem Behälter verklumpt, wodurch es ebenfalls zu erheblichen Variationen der durch das Druckgas geförderten Menge des pulverförmigen Mediums kommt.

Die Patentanmeldung US 2005/0267628 A1 offenbart eine Vorrichtung zur Abgabe von trockenen, pulverförmigen Substanzen, die in einem Behälter gelagert sind. Zur Förderung der Substanzen aus dem Behälter wird dieser mit Schwingungen beaufschlagt, wodurch die Förderung des Pulvers aus dem Behälter durch die Schwerkraft unterstützt wird. Gemäß einem Ausführungsbeispiel ist zusätzlich vorgesehen, ein Gas durch einen gaspermeablen Abschnitt in den an den Behälter anschließenden Förderweg des Pulvers zu leiten, damit das Gas das Pulver verdichtet, wodurch die Förderung des Pulvers durch die Gravitation verbessert werden soll.

Der vorliegenden Erfindung liegt daher die Aufgabe zugrunde, eine dentalmedizinische Vorrichtung zur Abgabe eines pulverförmigen Mediums zu schaffen, mit der ein konstanterer Betrieb möglich ist, insbesondere die Beladung des Druckgases mit dem pulverförmigen Medium weniger variiert und bei der eine Verklumpung des pulverförmigen Mediums in dem Behälter im Vergleich zur derzeitigen Praxis geringere Auswirkung auf die Förderung des pulverförmigen Mediums zu der Abgabeeinheit hat.

Diese Aufgabe wird gemäß der vorliegenden Erfindung durch eine dentalmedizinische Vorrichtung zur Abgabe eines pulverförmigen Mediums mit den Merkmalen des Anspruchs 1 gelöst. Besonders vorteilhafte Ausführungsformen sind in den Unteransprüchen angeführt.

Die dentalmedizinische Vorrichtung zur Abgabe eines pulverförmigen Mediums auf menschliches oder tierisches Gewebe oder auf ein Gewebe-Ersatzmaterial weist einen Behälter zum Aufbewahren des pulverförmigen Mediums in einem Behälterinnenraum auf, wobei der Behälter an eine Druckgasquelle anschließbar ist und Druckgas der Druckgasquelle in den Behälterinnenraum leitbar ist, um das pulverförmige Medium mit dem Druckgas aus dem Behälterinnenraum zu fördern. Der Behälter ist mit einer Abgabeeinheit verbindbar, so dass das pulverförmige Medium mit dem Druckgas an die Abgabeeinheit gefördert und von der Abgabeeinheit in Richtung des menschlichen oder tierischen Gewebes oder des Gewebe-Ersatzmaterials abgegeben werden kann. Der Behälter umfasst eine Behälterwand, die zumindest einen Teil des Behälterinnenraums definiert, und einen mit der Behälterwand verbundenen Behälterboden mit zumindest einer Bohrung, durch die Druckgas der Druckgasquelle in den Behälterinnenraum leitbar ist. Am Behälterboden ist des Weiteren eine druckgasdurchlässige Wirbelplatte aus porösem Material vorgesehen, wobei durch dieses poröse Material Druckgas in den Behälterinnenraum leitbar ist, um zumindest einen Teil des pulverförmigen Mediums in dem Behälterinnenraum aufzulockern, ohne das pulverförmige Medium aus dem Behälterinnenraum an die Abgabeeinheit zu fördern.

Das Vorsehen einer druckgasdurchlässigen Wirbelplatte aus porösem Material, durch das Druckgas in den Behälterinnenraum leitbar ist, bewirkt ein Auflockern und / oder Schweben des pulverförmigen Mediums in dem Behälterinnenraum. Alternativ kann die Wirkung des durch das poröse Material geblasenen Druckgases damit beschrieben werden, dass das Druckgas das pulverförmige Medium in einen flüssigkeitsähnlichen oder bewegungsaktiven, d.h. fluidisierten, Zustand versetzt. Dieses Auflockern oder Fluidisieren ermöglicht ein gleichbleibendes Beladen des Druckgases mit dem pulverförmigen Medium und wirkt auch dem Verklumpen des pulverförmigen Mediums entgegen und / oder trennt miteinander verklumpte Partikel des pulverförmigen Mediums voneinander. Die dentalmedizinische Vorrichtung ist somit mit erheblich konstanteren Betriebsparametern betreibbar.

Vorzugsweise wird als Druckgas Druckluft verwendet, welche die dentalmedizinische Vorrichtung zur Abgabe eines pulverförmigen Mediums von einer Druckluftquelle, zum Beispiel einem Kompressor, erhält. Dazu ist die dentalmedizinische Vorrichtung zur Abgabe eines pulverförmigen Mediums über eine Druckgasleitung/Druckluftleitung mit der Druckluftquelle verbunden.

Wie im Folgenden noch im Detail beschrieben wird, weist der Behälter ein Steigrohr auf, das sich in den Behälterinnenraum erstreckt und das zumindest eine Ansaugöffnung zum Ansaugen von in dem Behälter aufbewahrten pulverförmigen Medium hat, um das pulverförmige Medium durch das Steigrohr zu der Abgabeeinheit zu fördern. Die zumindest eine Ansaugöffnung und die druckgasdurchlässige Wirbelplatte sind nahe aneinander oder aneinander angrenzend angeordnet, so dass das Auflockern oder Fluidisieren des pulverförmigen Mediums insbesondere in einem Volumen an der zumindest einen Ansaugöffnung erfolgt, wodurch das Ansaugen des pulverförmigen Mediums in das Steigrohr zusätzlich begünstigt wird.

Vorzugsweise wird das gesamte in dem Behälter aufbewahrte pulverförmige Medium durch das Druckgas, das durch das poröse Material der druckgasdurchlässigen Wirbelplatte in den Behälterinnenraum gelangt, aufgelockert oder fluidisiert.

Die druckgasdurchlässige Wirbelplatte ist vorzugsweise aus Glas, Metall oder Kunststoff hergestellt. Die druckgasdurchlässige Wirbelplatte ist vorzugsweise als zylindrische Scheibe ausgebildet. Die druckgasdurchlässige Wirbelplatte weist vorzugsweise eine dem Behälterinnenraum zugewandte Oberseite, eine vom Behälterinnenraum entfernte Unterseite und einen sich zwischen der Oberseite und der Unterseite erstreckenden Körper mit einer Mantel- oder Seitenfläche auf. Die druckgasdurchlässige Wirbelplatte, insbesondere deren Körper, umfasst vorzugsweise eine Vielzahl von Kanälen oder Poren, welche insbesondere die Oberseite und die Unterseite verbinden und in Öffnungen an der Oberseite und Unterseite münden, so dass die druckgasdurchlässigen Wirbelplatte für Druckgas durchlässig ist. Der Durchmesser oder die Größe der Kanäle oder Poren ist vorzugsweise gleich oder kleiner 20 µm, insbesondere gleich oder kleiner 14 µm, so dass Partikel des pulverförmigen Mediums im Wesentlichen nicht in die Poren oder Kanäle eindringen oder durch diese hindurchtreten können.

Vorzugsweise ist die druckgasdurchlässige Wirbelplatte, insbesondere aufgrund der Vielzahl ihrer Kanäle oder Poren, derart ausgebildet, dass der druckgasdurchlässigen Wirbelplatte zugeleitetes Druckgas an zumindest einem Großteil ihrer Oberseite austritt, wodurch insbesondere das Auflockern oder Fluidisieren des pulverförmigen Mediums bewirkt wird, ohne das pulverförmige Medium aus dem Behälterinnenraum an die Abgabeeinheit zu fördern.

Bevorzugt umfasst der Behälterinnenraum zumindest einen Abschnitt oder ein Teilvolumen, der/das sich in Richtung des Behälterbodens verjüngt. Besonders bevorzugt ist die druckgasdurchlässige Wirbelplatte an oder in dem sich in Richtung des Behälterbodens verjüngenden Abschnitt des Behälterinnenraums angeordnet ist. Besonders bevorzugt ist die zumindest eine Ansaugöffnung des Steigrohrs in dem sich in Richtung des Behälterbodens verjüngenden Abschnitt des Behälterinnenraums angeordnet. Diese Merkmale bewirken in vorteilhafter Weise und insbesondere in Kombination ein zuverlässiges Fließen des pulverförmigen Mediums in dem Behälterinnenraum, ein zuverlässiges Einsaugen durch die Ansaugöffnungen des Steigrohrs und/oder wirken einer Brückenbildung des pulverförmigen Mediums entgegen.

Bevorzugt bildet die druckgasdurchlässige Wirbelplatte einen den Behälterinnenraum begrenzenden Zwischenboden, der den Behälterinnenraum von dem Behälterboden trennt. Damit ist in vorteilhafter Weise sichergestellt, dass das pulverförmige Medium ausschließlich auf der druckgasdurchlässigen Wirbelplatte (und nicht direkt auf dem Behälterboden) lagert und damit zuverlässig aufgelockert oder fluidisiert wird.

Bevorzugt ist unterhalb der druckgasdurchlässigen Wirbelplatte, insbesondere zwischen der druckgasdurchlässigen Wirbelplatte und dem Behälterboden, eine sich unmittelbar entlang der Unterseite der druckgasdurchlässigen Wirbelplatte, vorzugsweise großflächig, erstreckende Druckgaskammer zur flächigen Versorgung der druckgasdurchlässigen Wirbelplatte mit Druckgas vorgesehen. Vorzugsweise erstreckt sich die Druckgaskammer über zumindest 50%, besonders bevorzugt über zumindest 75%, der Unterseite der druckgasdurchlässigen Wirbelplatte. Vorzugsweise ist die Druckgaskammer als Ringkammer unterhalb der scheibenförmigen, druckgasdurchlässigen Wirbelplatte ausgebildet.

Vorzugsweise ist die druckgasdurchlässige Wirbelplatte von dem Behälterboden durch eine Schulter oder einen Abstandhalter beabstandet, wobei die Druckgaskammer insbesondere den durch die Schulter oder den Abstandhalter gebildeten Abstand umfasst.

Vorzugsweise mündet eine Druckgasleitung, die im Folgenden insbesondere als erste Druckgasleitung zur (ausschließlichen) Versorgung des porösen Materials der druckgasdurchlässigen Wirbelplatte mit Druckgas bezeichnet ist, in die Druckgaskammer. Vorzugsweise ist die Druckgaskammer ausgebildet und vorgesehen, das von der Druckgasleitung gelieferte Druckgas aufzunehmen und insbesondere flächig an der Unterseite der druckgasdurchlässigen Wirbelplatte zu verteilen, so dass das Druckgas an einer Vielzahl von Stellen der Unterseite in das poröse Material der druckgasdurchlässigen Wirbelplatte eindringt.

Vorzugsweise weist die druckgasdurchlässige Wirbelplatte eine, insbesondere zentral in der druckgasdurchlässigen Wirbelplatte angeordnete, Bohrung auf, durch die Druckgas der Druckgasquelle in den Behälterinnenraum leitbar ist, um das pulverförmige Medium aus dem Behälterinnenraum zu fördern. Die Bohrung bildet somit insbesondere einen Teil einer Leitung oder nimmt eine Leitung auf, die Druckgas direkt in den Behälterinnenraum leitet, ohne dass das Druckgas durch die Kanäle oder Poren der druckgasdurchlässigen Wirbelplatte fließen muss. Vorzugsweise hat die Bohrung einen Durchmesser von mehreren Millimetern.

Vorzugsweise umfasst die dentalmedizinische Vorrichtung, insbesondere der Behälter, eine erste Druckgasleitung und eine zweite Druckgasleitung. Die erste Druckgasleitung ist zur (ausschließlichen) Versorgung des porösen Materials der druckgasdurchlässigen Wirbelplatte mit Druckgas, vorzugsweise über die sich entlang der Unterseite der druckgasdurchlässigen Wirbelplatte erstreckenden Druckgaskammer vorgesehen, so dass das durch die erste Druckgasleitung geförderte Druckgas (ausschließlich) durch das poröse Material der druckgasdurchlässigen Wirbelplatte in den Behälterinnenraum des Behälters gelangt, um zumindest einen Teil des pulverförmigen Mediums in dem Behälterinnenraum aufzulockern. Die zweite Druckgasleitung ist zur Leitung von Druckgas (ausschließlichen) direkt in den Behälterinnenraum zum Fördern des pulverförmigen Mediums mit dem Druckgas aus dem Behälterinnenraum vorgesehen. Das in der zweiten Druckgasleitung geförderte Druckgas fließt somit insbesondere nicht durch die Poren und Kanäle des porösen Materials der druckgasdurchlässigen Wirbelplatte. Das in der zweiten Druckgasleitung geförderte Druckgas fließt besonders bevorzugt durch die zweite Druckgasleitung direkt, insbesondere durch die Bohrung der druckgasdurchlässigen Wirbelplatte, in den Behälterinnenraum oder in das Steigrohr.

Die erste Druckgasleitung und die zweite Druckgasleitung sind als separate, voneinander getrennte Druckgasleitungen ausgebildet. Durch das Vorsehen der zwei getrennten Druckgasleitung ist es vorteilhafter Weise möglich, Druckgas mit unterschiedlichen Parametern zur druckgasdurchlässigen Wirbelplatte und direkt in den Behälterinnenraum zu fördern, zum Beispiel unterschiedliche Druckgas-Volumenströme.

Vorzugsweise umfasst zumindest die erste Druckgasleitung oder die zweite Druckgasleitung ein Drosselelement, zum Beispiel ein Drosselventil, um den durch die erste Druckgasleitung oder die zweite Druckgasleitung strömenden Druckgas-Volumenstrom einstellen zu können. Besonders bevorzugt umfasst die erste Druckgasleitung ein erstes Drosselelement und die zweite Druckgasleitung ein zweites Drosselelement. Vorzugsweise ist das zumindest eine Drosselelement oder sind das erste Drosselelement und das zweite Drosselelement derart ausgebildet, dass der Druckgas-Volumenstrom nach dem Passieren des Drosselelements oder der Drosselelemente in der ersten Druckgasleitung und in der zweiten Druckgasleitung unterschiedlich sind, insbesondere derart, dass der Druckgas-Volumenstrom in der ersten Druckgasleitung (zur Versorgung der druckgasdurchlässigen Wirbelplatte) größer ist als in der zweiten Druckgasleitung. In mehreren Praxisversuchen wurde damit ein besonders gutes und konstantes Betriebsverhalten der dentalmedizinischen Vorrichtung, insbesondere eine gleichbleibende Beladung des Druckgases mit pulverförmigem Medium, erzielt.

Vorzugsweise erstreckt sich die zweite Druckgasleitung durch die Bohrung der druckgasdurchlässigen Wirbelplatte oder diese Bohrung ist Teil der zweiten Druckgasleitung. Damit ist eine besonders platzsparende Ausgestaltung geschaffen.

Vorzugsweise sind die Bohrung der druckgasdurchlässigen Wirbelplatte und / oder die druckgasdurchlässige Wirbelplatte und / oder zumindest ein Abschnitt der zweiten Druckgasleitung und / oder zumindest ein Abschnitt des Behälterinnenraums und / oder zumindest ein Abschnitt der Behälterwand konzentrisch angeordnet, insbesondere um eine Mittelachse des Behälters.

Vorzugsweise sind die erste Druckgasleitung und die zweite Druckgasleitung mit einer gemeinsamen Druckgasquelle verbindbar, insbesondere über eine gemeinsame Druckgasversorgungsleitung. Besonders bevorzugt ist die gemeinsame Druckgasversorgungsleitung mit einer Dentaleinheit mit einem Patientenstuhl verbindbar oder verbunden, so dass die dentalmedizinische Vorrichtung über oder durch die Dentaleinheit mit Druckgas versorgbar ist.

Vorzugsweise sind die erste Druckgasleitung und die zweite Druckgasleitung in einem mit dem Behälter verbindbaren Versorgungsblock angeordnet. Vorzugsweise ist der Versorgungsblock als Verbindungselement oder Anschlusselement zwischen dem Behälter und einem Basisteil oder Bedienteil der dentalmedizinischen Vorrichtung ausgebildet. Vorzugsweise verbindet der Versorgungsblock den Behälter lösbar oder unlösbar mit dem Basisteil oder Bedienteil. Vorzugsweise ist der Versorgungsblock aus Metall gefertigt, wobei die erste Druckgasleitung und die zweite Druckgasleitung als Bohrungen in dem Versorgungsblock ausgebildet sind.

Vorzugsweise ragt ein Fortsatz des Versorgungsblocks, in dem insbesondere ein Abschnitt der zweiten Druckgasleitung angeordnet ist, in die Bohrung der druckgasdurchlässigen Wirbelplatte, wodurch in vorteilhafter Weise eine Fixierung und Zentrierung der druckgasdurchlässigen Wirbelplatte, des Versorgungsblocks und des Behälters zueinander erfolgt. Vorzugsweise ist der Durchmesser oder die Breite des Fortsatzes des Versorgungsblocks geringer als der Durchmesser oder die Breite eines weiteren Abschnitts des Versorgungsblocks. Vorzugsweise ist in dem Fortsatz des Versorgungsblocks nur ein Abschnitt der zweiten Druckgasleitung angeordnet, wohingegen in dem weiteren Abschnitt des Versorgungsblocks ein Abschnitt der ersten Druckgasleitung und der zweiten Druckgasleitung angeordnet ist.

Vorzugsweise ist an der Schnittstelle zwischen dem Versorgungsblock, insbesondere dessen Fortsatz, und der druckgasdurchlässigen Wirbelplatte und / oder der Druckgaskammer und / oder dem Behälter, insbesondere dem Behälterboden, zumindest ein Dichtelement vorgesehen, um das Fließen von Druckgas entlang der Schnittstelle zu verhindern.

Vorzugsweise umfasst die dentalmedizinische Vorrichtung, insbesondere der Behälter, ein Steigrohr mit einem ersten Ende und einem zweiten Ende, wobei das Steigrohr sich in den Behälterinnenraum erstreckt. Das erste Ende des Steigrohrs schließt an die zweite Druckgasleitung und / oder an den Fortsatz des Versorgungsblocks an und / oder ragt in die Bohrung der druckgasdurchlässigen Wirbelplatte, so dass Druckgas aus der zweiten Druckgasleitung in das Steigrohr (und somit direkt in das Behälterinnere) fließen kann. Das Steigrohr weist des Weiteren zumindest eine, vorzugsweise mehrere, Ansaugöffnung zum Ansaugen von in dem Behälter aufbewahrten pulverförmigen Medium auf, so dass pulverförmiges Medium durch das von der zweiten Druckgasleitung gelieferte Druckgas durch die zumindest eine Ansaugöffnung in das Steigrohr gesaugt wird (insbesondere mittels Venturi-Effekt) und mit dem Druckgas durch das Steigrohr in Richtung des zweiten Endes gefördert wird.

Vorzugsweise erweitert sich der Innendurchmesser des Steigrohrs von der zumindest einen Ansaugöffnung in Richtung des zweiten Endes. Vorzugsweise sind drei oder mehr Ansaugöffnungen vorgesehen, die, insbesondere gleichmäßig, um den Umfang des Steigrohrs angeordnet sind. Die zumindest eine Ansaugöffnung ist an dem ersten Ende des Steigrohrs, nahe der druckgasdurchlässigen Wirbelplatte, angeordnet. Damit ist die zumindest eine Ansaugöffnung in einem Teilvolumen des Behälterinnenraums vorgesehen, in dem die Aufwirbelung oder Fluidisierung des pulverförmigen Mediums besonders intensiv ist, wodurch ein besonders gutes und konstantes Betriebsverhalten der dentalmedizinischen Vorrichtung, insbesondere eine gleichbleibende Beladung des Druckgases mit pulverförmigem Medium, erzielt wird.

Vorzugsweise umfasst die medizinische oder dentalmedizinische Vorrichtung, insbesondere der Behälter ein Rückschlagventil, das ausgebildet ist, das Eindringen von pulverförmigen Medium aus dem Behälterinnenraum in die zweite Druckgasleitung zu verhindern. Das Rückschlagventil umfasst zum Beispiel ein Entenschnabelventil.

Vorzugsweise ist zumindest ein Teil des Rückschlagventils in der zweiten Druckgasleitung, insbesondere in dem Versorgungsblock oder in dem Fortsatz des Versorgungsblocks angeordnet. Vorzugsweise durchsetzt das Rückschlagventil die Bohrung der druckgasdurchlässigen Wirbelplatte. Vorzugsweise ragt zumindest ein Teil des Rückschlagventils in das Steigrohr.

Vorzugsweise ist das Rückschlagventil durch das in der zweiten Druckgasleitung förderbare Druckgas derart betätigbar, dass das Rückschlagventil öffnet, wenn der Gasdruck des in der zweiten Druckgasleitung fließenden Druckgases einen vorbestimmten Wert übersteigt, und schließt, wenn der Gasdruck des in der zweiten Druckgasleitung fließenden Druckgases einen vorbestimmten Wert unterschreitet.

Vorzugsweise umfasst der Behälter, insbesondere die Behälterwand, eine Öffnung, durch die das mit dem pulverförmigen Medium beladene Druckgas aus dem Behälter in Richtung der Abgabeeinheit förderbar ist. Vorzugsweise ist die Öffnung mit einem Förderrohr verbunden und / oder das Förderrohr ist in der Öffnung aufgenommen, wobei das Förderrohr ausgebildet ist, das mit dem pulverförmigen Medium beladene Druckgas in Richtung der Abgabeeinheit zu fördern. Vorzugsweise sind die Öffnung oder das Förderrohr fluidleitend mit dem Steigrohr verbunden. Vorzugsweise ist der Innendurchmesser des Förderrohrs geringer als der Innendurchmesser des Steigrohrs. Vorzugsweise ist das Förderrohr mit einer Leitung verbunden, die das mit dem pulverförmigen Medium beladene Druckgas in Richtung der Abgabeeinheit fördert. Vorzugsweise erstreckt sich diese Leitung bis zu der Abgabeeinheit oder in diese hinein.

Vorzugsweise umfasst die dentalmedizinische Vorrichtung des Weiteren zumindest eines der folgenden Elemente: eine Abgabeeinheit, insbesondere einen Handgriff, mit einer Düsenvorrichtung, die zur Abgabe des pulverförmigen Mediums in Richtung des menschlichen oder tierischen Gewebes oder des Gewebe-Ersatzmaterials ausgebildet ist; eine Flüssigkeitsleitung zum Leiten einer Flüssigkeit in Richtung der Abgabeeinheit, so dass die Flüssigkeit über die Abgabeeinheit in Richtung des menschlichen oder tierischen Gewebes oder des Gewebe-Ersatzmaterials abgebbar ist; ein Basisteil oder Bedienteil, in dem zum Beispiel zumindest ein Abschnitt der ersten Druckgasleitung und / oder der zweiten Druckgasleitung und / oder der gemeinsamen Druckgasversorgungsleitung und / oder ein Feuchtigkeitsabscheider zum Abscheiden von Feuchtigkeit aus dem Druckgas, das dem Behälter zugeleitet wird, und / oder zumindest ein Abschnitt der Flüssigkeitsleitung und / oder zumindest ein Stellelement zum Stellen des Drosselelements für das Druckgas und / oder zumindest ein Stellelement zum Stellen der Flüssigkeits-Durchflussmenge durch die Flüssigkeitsleitung und / oder der Versorgungsblock und / oder eine Aufnahme für den Behälter vorgesehen sind; einen ersten Versorgungsschlauch mit einer Druckgasleitung zum Leiten von Druckgas von einer Druckgasquelle zu dem Basisteil oder Bedienteil oder dem Behälter und vorzugsweise mit einer Flüssigkeitsleitung zum Leiten einer Flüssigkeit von einer Flüssigkeitsquelle in Richtung der Abgabeeinheit; einen zweiten Versorgungsschlauch mit einer Druckgasleitung zum Leiten des mit dem pulverförmigen Medium beladenen Druckgases von dem Basisteil oder Bedienteil oder dem Behälter in Richtung der Abgabeeinheit und vorzugsweise mit einer Flüssigkeitsleitung zum Leiten einer Flüssigkeit in Richtung der Abgabeeinheit; eine Druckgasquelle.

Im Folgenden ist ein Verfahren zum Betrieb einer dentalmedizinischen Vorrichtung zur Abgabe eines pulverförmigen Mediums auf menschliches oder tierisches Gewebe oder auf ein Gewebe-Ersatzmaterial beschrieben, wobei die dentalmedizinische Vorrichtung einen Behälter zum Aufbewahren des pulverförmigen Mediums in einem Behälterinnenraum aufweist. Der Behälter ist an eine Druckgasquelle anschließbar und Druckgas der Druckgasquelle ist in den Behälterinnenraum leitbar, um das pulverförmige Medium mit dem Druckgas aus dem Behälterinnenraum zu fördern. Der Behälter der dentalmedizinischen Vorrichtung ist des Weiteren mit einer Abgabeeinheit verbindbar, so dass das pulverförmige Medium mit dem Druckgas an die Abgabeeinheit gefördert und von der Abgabeeinheit in Richtung des menschlichen oder tierischen Gewebes oder des Gewebe-Ersatzmaterials abgegeben werden kann. Der Behälter weist insbesondere eine Behälterwand, die zumindest einen Teil des Behälterinnenraums definiert, und einen mit der Behälterwand verbundenen Behälterboden mit zumindest einer Bohrung auf, wobei durch die Bohrung Druckgas der Druckgasquelle in den Behälterinnenraum leitbar ist. An dem Behälterboden ist eine druckgasdurchlässige Wirbelplatte aus porösem Material vorgesehen. Das Verfahren umfasst den Schritt, dass durch das poröse Material der druckgasdurchlässigen Wirbelplatte Druckgas in den Behälterinnenraum geleitet wird, um zumindest einen Teil des pulverförmigen Mediums in dem Behälterinnenraum aufzulockern oder zu fluidisieren, ohne das pulverförmige Medium aus dem Behälterinnenraum an die Abgabeeinheit zu fördern.

Vorzugsweise wird bei dem Verfahren des Weiteren Druckgas durch das im Vorstehenden bereits beschriebene Steigrohr geleitet, um das pulverförmige Medium in das Steigrohr zu saugen, bevorzugt mittels Venturi-Effekt, und insbesondere durch das Steigrohr zu der Abgabeeinheit zu fördern.

Vorzugsweise werden das Steigrohr und die druckgasdurchlässige Wirbelplatte mit zwei getrennten Druckgasströmen versorgt, die ihnen insbesondere über die im Vorstehenden bereits beschriebene erste Druckgasleitung und zweite Druckgasleitung zugeleitet werden. Besonders bevorzugt weisen die zwei getrennten Druckgasströme unterschiedliche Volumenströme auf, die insbesondere mittels dem bereits beschriebenen, zumindest einem Drosselelement eingestellt werden.

Die Erfindung wird nachfolgend anhand bevorzugter Ausführungsbeispiele und Bezug nehmend auf die beigefügten Zeichnungen erläutert. Es zeigt die
Figur 1 eine dentalmedizinische Vorrichtung zur Abgabe eines pulverförmigen Mediums;
Figur 2 einen an eine Druckgasquelle anschließbaren Behälter zum Aufbewahren des pulverförmigen Mediums;
Figur 3 den in der Figur 2 mit "A" gekennzeichneten Ausschnitt.

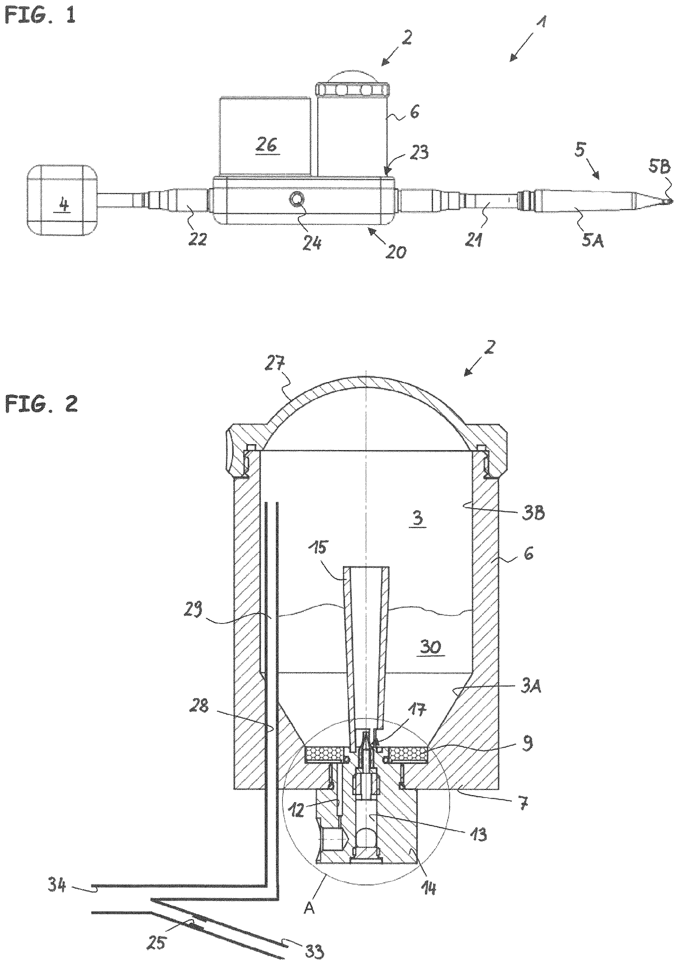

Die in der Figur 1 dargestellte dentalmedizinische Vorrichtung 1 zur Abgabe eines pulverförmigen Mediums 30 auf menschliches oder tierisches Gewebe oder auf ein Gewebe-Ersatzmaterial umfasst einen Behälter 2 zum Aufbewahren des pulverförmigen Mediums 30, ein Basisteil oder Bedienteil 20, eine Abgabeeinheit 5, einen ersten Versorgungsschlauch 21, der das Basisteil oder Bedienteil 20 mit der Abgabeeinheit 5 verbindet und einen zweiten Versorgungsschlauch 22 zur Verbindung des Basisteils oder Bedienteils 20 mit einer Druckgasquelle und / oder Dentaleinheit 4.

Die Druckgasquelle ist entweder als eigenständiges Element, das mit der Dentaleinheit 4 verbunden ist, oder als Teil der Dentaleinheit 4 ausgebildet.

Die Dentaleinheit 4 umfasst neben der Druckgasquelle oder einer Verbindung dazu vorzugsweise einen Dentalstuhl, eine Verbindung zu einer Flüssigkeitsquelle, eine Steuervorrichtung zur Steuerung einer Vielzahl unterschiedlicher dentaler Instrumente, insbesondere Handstück, sowie mehrere Anschlüsse, an welche die dentalen Instrumente anschließbar sind, um von der Dentaleinheit 4 Medien wie Flüssigkeit (Wasser), Druckgas, insbesondere Druckluft, und / oder elektrische Energie zu erhalten. Bevorzugt ist die dentalmedizinische Vorrichtung 1 zur Abgabe eines pulverförmigen Mediums 30 mit der Dentaleinheit 4 über einen dieser Anschlüsse verbindbar, um zumindest Druckgas, gegebenenfalls auch Wasser und / oder elektrische Energie, für den Betrieb der dentalmedizinische Vorrichtung 1 zu empfangen.

Die Abgabeeinheit 5 umfasst zumindest eine Düsenvorrichtung 5B zur Abgabe des pulverförmigen Mediums 30 in Richtung des menschlichen oder tierischen Gewebes oder des Gewebe-Ersatzmaterials. Vorzugsweise umfasst die Abgabeeinheit 5 einen mit einer Hand haltbaren Handgriff 5A, an dem die Düsenvorrichtung 5B befestigt ist. Eine Leitung, die das mit dem pulverförmigen Medium 30 beladene Druckgas fördert, ist mit dem Handgriff 5A und / oder der Düsenvorrichtung 5B verbunden und / oder erstreckt sich in den Handgriff 5A und / oder die Düsenvorrichtung 5B.

Der zweite Versorgungsschlauch 22 umfasst zumindest eine Leitung zum Leiten von Druckgas von der Druckgasquelle und / oder Dentaleinheit 4 zu dem Basisteil oder Bedienteil 20 und / oder in Richtung der Abgabeeinheit 5. Der erste Versorgungsschlauch 21 umfasst zumindest eine Leitung zum Leiten von mit dem pulverförmigen Medium 30 beladenem Druckgas von dem Behälter 2 und / oder dem Basisteil oder Bedienteil 20 in Richtung der Abgabeeinheit 5, insbesondere der Düsenvorrichtung 5B. Vorzugsweise umfassen der erste Versorgungsschlauch 21 und / oder der zweite Versorgungsschlauch 22 zusätzlich eine Leitung zum Leiten einer Flüssigkeit in Richtung der Abgabeeinheit 5 und / oder eine elektrische Leitung zum Leiten elektrischer Energie zu dem Basisteil oder Bedienteil 20 oder in Richtung der Abgabeeinheit 5.

An dem Basisteil oder Bedienteil 20 ist der Behälter 2 vorgesehen, zum Beispiel lösbar oder unlösbar daran befestigt. Vorzugsweise ist an dem Basisteil oder Bedienteil 20 eine Aufnahme 23 für den Behälter 2 und / oder eine Anschlussvorrichtung zur Leitung von Druckgas von dem Basisteil oder Bedienteil 20 oder einer darin angeordneten Druckgasleitung in den Behälter 2 und / oder von dem Behälter 2 in den Basisteil oder Bedienteil 20 oder eine darin angeordnete Druckgasleitung vorgesehen.

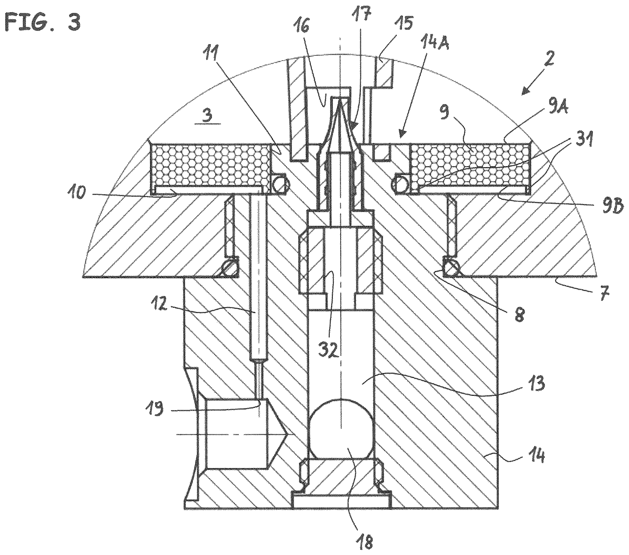

Neben den im Vorstehenden bereits erwähnten Druckgasleitungen und Flüssigkeitsleitungen kann in dem Basisteil oder Bedienteil 20 vorzugsweise auch zumindest ein Stellelement vorgesehen sein, zum Beispiel zumindest ein Drosselelement 18, 19 oder 25 (siehe Fig. 2 oder 3) in einer Druckgasleitung oder zumindest ein Ventil zum Verändern der Durchflussmenge einer Flüssigkeit in einer Flüssigkeitsleitung, die Flüssigkeit in Richtung der Abgabeeinheit 5 leitet. Vorzugsweise ist an dem Basisteil oder Bedienteil 20 zumindest ein Bedienelement 24 vorgesehen, mit dem ein Anwender ein in dem Basisteil oder Bedienteil 20 vorgesehenes Stellelement bedienen kann, zum Beispiel das im Vorstehenden genannte Ventil zum Verändern der Durchflussmenge einer Flüssigkeit oder zumindest eines der im Vorstehenden genannten Drosselelemente.

In dem Basisteil oder Bedienteil 20 ist vorzugsweise auch ein Versorgungsblock 14 (Fig. 2) vorgesehen, der den Behälter 2 mit dem Basisteil oder Bedienteil 20 und insbesondere mit zumindest einer der in dem Basisteil oder Bedienteil 20 vorgesehenen Druckgasleitungen verbindet. Der im Nachstehenden noch detaillierter beschriebene Versorgungsblock 14 ist auch zur mechanischen Verbindung des Behälters 2 mit dem Basisteil oder Bedienteil 20 ausgebildet.

Schließlich ist in dem Basisteil oder Bedienteil 20 vorzugsweise ein Feuchtigkeitsabscheider 26 zum Abscheiden von Feuchtigkeit aus dem Druckgas, das dem Behälter 2 zugeleitet wird, angeordnet. Der Feuchtigkeitsabscheider 26 umfasst zum Beispiel Filtermaterial oder ein Trocknungsmittel.

Der in Figur 2 vergrößert dargestellte Behälter 2 umfasst eine, vorzugsweise zylindrische, Behälterwand 6, die zumindest einen Teil eines Behälterinnenraums 3 definiert, einen mit der Behälterwand 6 verbundenen Behälterboden 7 und einen Behälterdeckel 27.

Der Behälter 2 ist über zumindest einen Anschluss an die Druckgasquelle 4 anschließbar, so dass Druckgas der Druckgasquelle 4 in den Behälterinnenraum 3 leitbar ist. Der Behälter 2 ist des Weiteren mit der Abgabeeinheit 5 verbindbar, so dass das mit dem pulverförmigen Medium 30 beladene Druckgas an die Abgabeeinheit 5 gefördert werden kann.

Der Behälterdeckel 27 ist vorzugsweise gegenüber dem Behälterboden 7 angeordnet. Der Behälterdeckel 27 ist vorzugsweise relativ zu dem Behälter 2, insbesondere zu Behälterwand 6, beweglich angeordnet oder davon lösbar, sodass ein pulverförmiges Medium 30 in den Behälterinnenraum 3 gefüllt werden kann. Der Behälterdeckel 27 hat vorzugsweise eine kuppelartige oder von dem Behälterinnenraum 3 nach außen gewölbte Form.

Der Behälterinnenraum 3 umfasst bevorzugt zumindest einen zylindrischen Abschnitt 3B. Vorzugsweise schließt an diesen zylindrischen Abschnitt 3B ein sich, insbesondere in Richtung des Behälterbodens 7, verjüngender Abschnitt 3A an.

Am, vorzugsweise kreisrunden, Behälterboden 7 ist zumindest eine Bohrung 8 (siehe Fig. 3) vorgesehen, durch die Druckgas der Druckgasquelle 4 in den Behälterinnenraum 3 leitbar ist. Am Behälterboden 7 ist vorzugsweise eine weitere Bohrung 28 vorgesehen, durch die das mit dem pulverförmigen Medium 30 beladene Druckgas den Behälterinnenraum 3 verlassen kann. Diese Bohrung 28 kann zumindest teilweise oder auch vollständig in der Behälterwand 6 und / oder in dem Behälterdeckel 27 vorgesehen sein.

Vorzugsweise ist ein Förderrohr 29 in der Bohrung 28 aufgenommen oder damit verbunden. Das Förderrohr 29 ist ausgebildet, das mit dem pulverförmigen Medium 30 beladene Druckgas aus dem Behälterinnenraum 3 zu leiten, insbesondere in Richtung der Abgabeeinheit 5. Das Förderrohr 29 ist dazu insbesondere mit einer in dem ersten Versorgungsschlauch vorgesehenen Druckgasleitung verbunden.

An dem Behälterboden 7 ist eine druckgasdurchlässige Wirbelplatte 9 aus porösem Material vorgesehen, wobei durch das poröse Material Druckgas in den Behälterinnenraum 3 leitbar ist, um zumindest einen Teil des pulverförmigen Mediums 30 in dem Behälterinnenraum 3 aufzulockern, ohne das pulverförmige Medium 30 aus dem Behälterinnenraum 3 an die Abgabeeinheit 5 zu fördern. Die druckgasdurchlässige Wirbelplatte 9 ist vorzugsweise kreisrund oder flachzylindrisch ausgebildet. Die Höhe der druckgasdurchlässigen Wirbelplatte 9 beträgt vorzugsweise mehrere Millimeter, zum Beispiel weniger als 7 mm, insbesondere weniger als 5 mm. Der Durchmesser der druckgasdurchlässigen Wirbelplatte 9 beträgt vorzugsweise weniger als 50 mm, insbesondere weniger als 35 mm. Im Körper der druckgasdurchlässige Wirbelplatte 9 sind eine Vielzahl von Kanälen oder Poren vorgesehen, die an der Oberseite 9A und an der Unterseite 9B der druckgasdurchlässige Wirbelplatte 9 in Öffnungen münden, so dass Druckgas durch die druckgasdurchlässigen Wirbelplatte 9 strömen kann.

Die druckgasdurchlässige Wirbelplatte 9 ist insbesondere anschließend an oder in dem sich in Richtung des Behälterbodens 7 verjüngenden Abschnitt 3A des Behälterinnenraums 3 angeordnet, so dass insbesondere das pulverförmigen Medium 30 in Richtung oder auf die druckgasdurchlässige Wirbelplatte 9 gleitet. Besonders bevorzugt endet der sich verjüngende Abschnitt 3A mit seinem geringsten Durchmesser an der druckgasdurchlässigen Wirbelplatte 9, insbesondere an deren Oberseite 9A (siehe auch Fig. 3), so dass das (gesamte) in dem Behälterinnenraum 3 aufbewahrte pulverförmigen Medium 30 unmittelbar auf der druckgasdurchlässige Wirbelplatte 9 lagert.

Vorzugsweise ist die druckgasdurchlässige Wirbelplatte 9 unlösbar in dem Behälter 2 befestigt, zum Beispiel in den aus Kunststoff gefertigten Behälter 2 eingespritzt.

Wie insbesondere in der Figur 2 gut zu erkennen ist, bildet die druckgasdurchlässige Wirbelplatte 9 einen den Behälterinnenraum 3 begrenzenden Zwischenboden, der den Behälterinnenraum 3 und / oder das pulverförmige Medium 30 von dem Behälterboden 7 trennt. Die druckgasdurchlässige Wirbelplatte 9 oder der Zwischenboden ist bevorzugt in dem Behälterinnenraum 3 oder daran angrenzend, insbesondere in einem zylindrisch geformten Aufnahmeraum, angeordnet.

Die druckgasdurchlässige Wirbelplatte 9 weist des Weiteren eine, vorzugsweise zentral angeordnete, Bohrung 11 auf, so dass Druckgas der Druckgasquelle 4 (direkt, ohne Durchfließen des porösen Materials) in den Behälterinnenraum 3 leitbar ist, um das pulverförmige Medium 30 aus dem Behälterinnenraum 3 zu fördern.

Wie insbesondere in der Figur 3 zu erkennen ist, ist die druckgasdurchlässige Wirbelplatte 9 von dem Behälterboden durch zumindest eine Schulter oder einen Abstandhalter 31 beabstandet, zum Beispiel durch einen oder gemäß Figur 3 zwei ringförmige(n) Abstandhalter. Dadurch ist unterhalb der druckgasdurchlässigen Wirbelplatte 9, insbesondere zwischen der druckgasdurchlässigen Wirbelplatte 9 und dem Behälterboden 7, eine sich großflächig entlang einer Unterseite 9B der druckgasdurchlässigen Wirbelplatte 9 erstreckenden Druckgaskammer 10 gebildet. Diese Druckgaskammer 10 ist zur flächigen Versorgung der druckgasdurchlässigen Wirbelplatte 9 mit Druckgas vorgesehen.

Die Druckgaskammer 10 ist über eine erste Druckgasleitung 12 mit der Druckgasquelle 4 verbunden, so dass Druckgas in die Druckgaskammer 10 förderbar ist. Das Druckgas verteilt sich in der Druckgaskammer 10 flächig unter der druckgasdurchlässigen Wirbelplatte 9, insbesondere unter deren Unterseite 9B, und fließt anschließend durch das poröse Material der druckgasdurchlässigen Wirbelplatte 9 in den Behälterinnenraum 3. Der Druckabfall des Druckgases über die druckgasdurchlässige Wirbelplatte 9 und / oder der in die Druckgaskammer 10 gelangende Volumenstrom des Druckgases bewirkt / bewirken, dass das in dem Behälterinnenraum 3 aufbewahrte pulverförmige Medium 30 durch das Druckgas, welches durch die druckgasdurchlässige Wirbelplatte 9 fließt, aufgelockert, aufgewirbelt oder fluidisiert wird, ohne pulverförmiges Medium 30 aus dem Behälterinnenraum 3 an die Abgabeeinheit 5 zu fördern.

Eine zweite Druckgasleitung 13 leitet Druckgas direkt in den Behälterinnenraum 3, um das pulverförmige Medium 30 mit dem Druckgas aus dem Behälterinnenraum 3 zu fördern. Dazu ist die zweite Druckgasleitung 13 ebenfalls mit der Druckgasquelle 4 verbunden. Bevorzugt sind die erste Druckgasleitung 12 und die zweite Druckgasleitung 13 über eine gemeinsame, in dem zweiten Versorgungsschlauch 22 angeordnete Druckgasleitung mit der Druckgasquelle 4 verbunden. Vorzugsweise teilt sich diese gemeinsame Druckgasleitung in dem Basisteil oder Bedienteil 20 in die erste Druckgasleitung 12 und die zweite Druckgasleitung 13 auf, so dass die beiden Druckgasleitungen 12, 13 anschließend als separate, voneinander getrennte Druckgasleitungen ausgebildet sind.

Die zweite Druckgasleitung 13 erstreckt sich durch die Bohrung 11 der druckgasdurchlässigen Wirbelplatte 9 und endet an oder in dem Behälterinnenraum 3. Ein Steigrohr 15 schließt an dieses Ende der zweiten Druckgasleitung 13 an oder ist damit verbunden.

Das Steigrohr 15 erstreckt sich weiter in den Behälterinnenraum 3 bis zu einem freien, zweiten Ende, das in dem Behälterinnenraum 3 oberhalb des pulverförmigen Mediums 30 angeordnet ist. Das Steigrohr 15 weist mehrere Ansaugöffnungen 16 zum Ansaugen von in dem Behälter 2 aufbewahrten pulverförmigen Medium 30 auf, so dass pulverförmiges Medium 30 durch das von der zweiten Druckgasleitung 13 gelieferte Druckgas durch die zumindest eine Ansaugöffnung 16 in das Steigrohr 15 gesaugt wird und mit dem Druckgas durch das Steigrohr 15 in Richtung des zweiten Endes gefördert wird.

Am zweiten, freien Ende tritt das Druckgas mit dem fluidisierten pulverförmigen Medium 30 aus dem Steigrohr 15 aus und fließt durch den Behälterinnenraum 3 zu dem Förderrohr 29, durch das es, wie im Vorstehenden bereits beschrieben, den Behälter 2 verlässt.

Die erste Druckgasleitung 12 und die zweite Druckgasleitung 13 sind in einem mit dem Behälter 2 verbindbaren Versorgungsblock 14 angeordnet. Der Versorgungsblock 14 ist als Verbindungselement oder Anschlusselement zwischen dem Behälter 2 und dem Basisteil oder Bedienteil 20 ausgebildet. Mehrere Dichtelemente dichten den Versorgungsblock 14 gegenüber dem Behälter 2, dem Behälterinnenraum 3, der druckgasdurchlässigen Wirbelplatte 9 und / oder der Druckgaskammer 10 ab.

Der Versorgungsblock 14 weist an einem Ende, insbesondere an dem dem Behälterinnenraum 3 zugewandten Ende, ein Fortsatz 14A auf. Dieser Fortsatz 14A ragt in die Bohrung 11 der druckgasdurchlässigen Wirbelplatte 9 und verschließt insbesondere die Bohrung 11. Der Fortsatz 14A, vorzugsweise dessen dem Behälterinnenraum 3 zugewandte Oberseite, bildet somit gemeinsam mit der druckgasdurchlässigen Wirbelplatte 9 insbesondere auch einen Teil des im Vorstehenden genannten Zwischenbodens.

In dem Fortsatz 14A ist ein Abschnitt der zweiten Druckgasleitung 13 angeordnet, der an der Oberseite des Fortsatzes 14A in eine Öffnung mündet, die mit dem Steigrohr 15 verbunden ist.

In der zweiten Druckgasleitung 13, insbesondere in dem Versorgungsblock 14 oder in dessen Fortsatz 14A, ist ein Rückschlagventil 17 angeordnet, das ausgebildet ist, das Eindringen von pulverförmigen Medium 30 aus dem Behälterinnenraum 3 in die zweite Druckgasleitung 13 zu verhindern. Wie aus der Figur 3 zu erkennen ist, ist das Rückschlagventil 17 an einer Schulter, die durch eine Verengung der zweiten Druckgasleitung 13 gebildet ist, gelagert und durch eine Hohlschraube 32 mit einer Innenbohrung an der Schulter fixiert.

Die erste Druckgasleitung 12 und die zweite Druckgasleitung 13 umfassen jeweils ein Drosselelement 18, 19, zum Beispiel ein Drosselventil, so dass die durch die erste Druckgasleitung 12 und die zweite Druckgasleitung 13 strömenden Druckgas-Volumenströme unabhängig voneinander einstellbar sind. Insbesondere sind die beiden Drosselelement 18, 19 derart ausgebildet oder betätigbar, zum Beispiel mit dem Bedienelement 24, dass der durch die erste Druckgasleitung 12 fließende Druckgas-Volumenstrom und der durch die zweite Druckgasleitung 13 fließende Druckgas-Volumenstrom unterschiedliche Werte aufweisen. Vorzugsweise ist der durch die erste Druckgasleitung 12 fließende Druckgas-Volumenstrom größer als der durch die zweite Druckgasleitung 13 fließende Druckgas-Volumenstrom.

Die dentalmedizinische Vorrichtung 1 umfasst bevorzugt eine weitere Druckgasleitung 33, die ausgebildet ist, reines Druckgas (ohne pulverförmigem Medium 30) in das Förderrohr 29 oder die Druckgasleitung 34 einzuleiten (siehe Fig. 2). Damit ist es möglich, die Konzentration oder Menge des fluidisierten, in Richtung der Abgabeeinheit 5 fließenden pulverförmigen Mediums 30 zu variieren, zum Beispiel durch Verdünnen des fluidisierten pulverförmigen Mediums 30 oder durch Erzeugen eines Staudrucks in dem Förderrohr 29. Damit kann auch das Fördern eines Pulverüberschusses beim Starten und Stoppen der Vorrichtung 1 in Richtung des Handgriffs 5A verhindert werden.

Die Druckgasleitung 33 ist mit der Druckgasquelle 4 verbunden und empfängt von dieser Druckgas ohne pulverförmigem Medium 30. Des Weiteren ist die Druckgasleitung 33 außerhalb des Behälters 2 mit dem Förderrohr 29 oder mit einer mit dem Förderrohr 29 verbundenen Druckgasleitung 34 zur Leitung des fluidisierten pulverförmigen Mediums 30 in Richtung der Abgabeeinheit 5 verbunden, so dass reines Druckgas in das Förderrohr 29 oder in die Druckgasleitung 34 förderbar ist.

Um die Konzentration oder Menge des fluidisierten, in Richtung der Abgabeeinheit 5 fließenden pulverförmigen Mediums 30 besonders gut variieren zu können, ist in der Druckgasleitung 33 ein weiteres Drosselelement 25 vorgesehen. Das Drosselelement 25 ist insbesondere stellbar ausgebildet, zum Beispiel mittels eines Bedienelements 24 an dem Basisteil oder Bedienteil 20.

Die Erfindung ist nicht auf die beschriebenen Ausführungsbeispiele beschränkt, sondern umfasst alle Ausführungen, die das prinzipielle, sinngemäße Funktionsprinzip der Erfindung gemäß den Ansprüchen anwenden oder beinhalten. Des Weiteren ist jedes beschriebene oder dargestellte Merkmal mit jedem anderen beschriebenen oder dargestellten Merkmal kombinierbar.

## Patentansprüche

1. Dentalmedizinische Vorrichtung (1) zur Abgabe eines pulverförmigen Mediums (30) auf menschliches oder tierisches Gewebe oder auf ein Gewebe-Ersatzmaterial mit einem Behälter (2) zum Aufbewahren des pulverförmigen Mediums (30) in einem Behälterinnenraum (3), wobei der Behälter (2) an eine Druckgasquelle (4) anschließbar ist und Druckgas der Druckgasquelle (4) in den Behälterinnenraum (3) leitbar ist, um das pulverförmige Medium (30) mit dem Druckgas aus dem Behälterinnenraum (3) zu fördern, und wobei der Behälter (2) mit einer Abgabeeinheit (5) verbindbar ist, so dass das pulverförmige Medium (30) mit dem Druckgas an die Abgabeeinheit (5) gefördert und von der Abgabeeinheit (5) in Richtung des menschlichen oder tierischen Gewebes oder des Gewebe-Ersatzmaterials abgegeben werden kann, wobei der Behälter (2) aufweist: eine Behälterwand (6), die zumindest einen Teil des Behälterinnenraums (3) definiert, und einen mit der Behälterwand (6) verbundenen Behälterboden (7) mit zumindest einer Bohrung (8), durch die Druckgas der Druckgasquelle (4) in den Behälterinnenraum (3) leitbar ist, wobei an dem Behälterboden (7) eine druckgasdurchlässige Wirbelplatte (9) aus porösem Material vorgesehen ist, wobei durch das poröse Material der druckgasdurchlässigen Wirbelplatte (9) Druckgas in den Behälterinnenraum (3) leitbar ist, um zumindest einen Teil des pulverförmigen Mediums (30) in dem Behälterinnenraum (3) aufzulockern, ohne das pulverförmige Medium (30) aus dem Behälterinnenraum (3) an die Abgabeeinheit (5) zu fördern, **gekennzeichnet durch** ein Steigrohr (15), das sich in den Behälterinnenraum (3) erstreckt und das zumindest eine Ansaugöffnung (16) zum Ansaugen von in dem Behälter (2) aufbewahrten pulverförmigen Medium (30) aufweist, um das pulverförmige Medium (30) durch das Steigrohr (15) zu der Abgabeeinheit (5) zu fördern, wobei die zumindest eine Ansaugöffnung (16) und die druckgasdurchlässigen Wirbelplatte (9) nahe aneinander angeordnet sind, so dass das Auflockern des pulverförmigen Mediums (30) an der zumindest einen Ansaugöffnung (16) erfolgt, wodurch das Ansaugen des pulverförmigen Mediums (30) in das Steigrohr (15) begünstigt ist.

2. Dentalmedizinische Vorrichtung (1) nach Anspruch 1, **dadurch gekennzeichnet, dass** die druckgasdurchlässige Wirbelplatte (9) an oder in einem sich in Richtung des Behälterbodens (7) verjüngenden Abschnitt (3A) des Behälterinnenraums (3) angeordnet ist.

3. Dentalmedizinische Vorrichtung (1) nach Anspruch 1 oder 2, **dadurch gekennzeichnet, dass**
die druckgasdurchlässige Wirbelplatte (9) einen den Behälterinnenraum (3) begrenzenden Zwischenboden bildet, der den Behälterinnenraum (3) von dem Behälterboden (7) trennt.

4. Dentalmedizinische Vorrichtung (1) nach einem der vorstehenden Ansprüche, **gekennzeichnet durch**
eine unterhalb der druckgasdurchlässigen Wirbelplatte (9), insbesondere zwischen der druckgasdurchlässigen Wirbelplatte (9) und dem Behälterboden (7), angeordnete, sich entlang einer Unterseite (9B) der druckgasdurchlässigen Wirbelplatte (9) erstreckenden Druckgaskammer (10) zur flächigen Versorgung der druckgasdurchlässigen Wirbelplatte (9) mit Druckgas.

5. Dentalmedizinische Vorrichtung (1) nach einem der vorstehenden Ansprüche, **dadurch gekennzeichnet, dass**
die druckgasdurchlässige Wirbelplatte (9) eine, vorzugsweise zentral angeordnete, Bohrung (11) aufweist, durch die Druckgas der Druckgasquelle (4) in den Behälterinnenraum (3) leitbar ist, um das pulverförmige Medium (30) aus dem Behälterinnenraum (3) zu fördern.

6. Dentalmedizinische Vorrichtung (1) nach einem der vorstehenden Ansprüche, **gekennzeichnet durch**
eine erste Druckgasleitung (12) zur Versorgung des porösen Materials der druckgasdurchlässigen Wirbelplatte (9) mit Druckgas, vorzugsweise über die sich entlang der Unterseite (9B) der druckgasdurchlässigen Wirbelplatte (9) erstreckenden Druckgaskammer (10), so dass das durch die erste Druckgasleitung (12) geförderte Druckgas durch das poröse Material der druckgasdurchlässigen Wirbelplatte (9) in den Behälterinnenraum (3) des Behälters (2) gelangt, um zumindest einen Teil des pulverförmigen Mediums (30) in dem Behälterinnenraum (3) aufzulockern, und eine zweite Druckgasleitung (13) zur Leitung von Druckgas direkt in den Behälterinnenraum (3) zum Fördern des pulverförmigen Mediums (30) mit dem Druckgas aus dem Behälterinnenraum (3), wobei die erste Druckgasleitung (12) und die zweite Druckgasleitung (13) als separate, voneinander getrennte Druckgasleitungen ausgebildet sind.

7. Dentalmedizinische Vorrichtung (1) nach Anspruch 6, **dadurch gekennzeichnet, dass** sich die zweite Druckgasleitung (13) durch die Bohrung (11) der druckgasdurchlässigen Wirbelplatte (9) erstreckt.

8. Dentalmedizinische Vorrichtung (1) nach Anspruch 6 oder 7, **dadurch gekennzeichnet, dass**
die erste Druckgasleitung (12) und die zweite Druckgasleitung (13) mit einer gemeinsamen Druckgasquelle (4) verbindbar sind.

9. Dentalmedizinische Vorrichtung (1) nach Anspruch 6, 7 oder 8, **dadurch gekennzeichnet, dass**
die erste Druckgasleitung (12) und die zweite Druckgasleitung (13) in einem mit dem Behälter (2) verbindbaren Versorgungsblock (14) angeordnet sind.

10. Dentalmedizinische Vorrichtung (1) nach Anspruch 9, **dadurch gekennzeichnet, dass** ein Fortsatz (14A) des Versorgungsblocks (14), in dem insbesondere ein Abschnitt der zweiten Druckgasleitung (13) angeordnet ist, in die Bohrung (11) der druckgasdurchlässigen Wirbelplatte (9) ragt.

11. Dentalmedizinische Vorrichtung (1) nach einem der Ansprüche 6 - 11, **gekennzeichnet durch**
ein Steigrohr (15) mit einem ersten Ende und einem zweiten Ende, wobei das erste Ende an die zweite Druckgasleitung (13) anschließt, so dass pulverförmiges Medium (30) durch das von der zweiten Druckgasleitung (13) gelieferte Druckgas durch die zumindest eine Ansaugöffnung (16) in das Steigrohr (15) gesaugt wird und mit dem Druckgas durch das Steigrohr (15) in Richtung des zweiten Endes gefördert wird.

12. Dentalmedizinische Vorrichtung (1) nach Anspruch 11, **dadurch gekennzeichnet, dass**
die dentalmedizinische Vorrichtung (1) des Weiteren eine Flüssigkeitsleitung zum Leiten einer Flüssigkeit in Richtung der Abgabeeinheit (5) aufweist, so dass die Flüssigkeit über die Abgabeeinheit (5) in Richtung des menschlichen oder tierischen Gewebes oder des Gewebe-Ersatzmaterials abgebbar ist.

13. Dentalmedizinische Vorrichtung (1) nach einem der Ansprüche 6 - 12, **gekennzeichnet durch**
ein Rückschlagventil (17), das ausgebildet ist, das Eindringen von pulverförmigen Medium (30) aus dem Behälterinnenraum (3) in die zweite Druckgasleitung (13) zu verhindern.

14. Dentalmedizinische Vorrichtung (1) nach einem der Ansprüche 6 - 13, **dadurch gekennzeichnet, dass**
zumindest die erste Druckgasleitung (12) oder die zweite Druckgasleitung (13) ein Drosselelement (18, 19) aufweisen, so dass der durch die erste Druckgasleitung (12) oder die zweite Druckgasleitung (13) strömende Druckgas-Volumenstrom einstellbar ist.

15. Dentalmedizinische Vorrichtung (1) nach einem der vorstehenden Ansprüche, **gekennzeichnet durch**
eine Abgabeeinheit (5), insbesondere einen Handgriff (5A), mit einer Düsenvorrichtung (5B), die zur Abgabe des pulverförmigen Mediums (30) in Richtung des menschlichen oder tierischen Gewebes oder des Gewebe-Ersatzmaterials ausgebildet ist.

## Claims

1. A dental device (1) for dispensing a powdered medium (30) onto human or animal tissue or onto a tissue replacement material with a container (2) for storing the powdered medium (30) in a container interior space (3), wherein the container (2) can be connected to a compressed gas source, and compressed gas from the compressed gas source (4) can be conducted into the container interior space (3) to convey the powdered medium (30) together with the compressed gas out of the container interior space (3), and wherein the container (2) can be connected to a dispensing unit (5) so that the powdered medium (30) can be conveyed with the compressed gas to the dispensing unit (5) and can be dispensed by the dispensing unit (5) in the direction of the human or animal tissue or the tissue replacement material, wherein the container (2) comprises: a container wall (6) which defines at least a portion of the container interior space (3) and a container bottom (7) that is connected to the container wall (6) and has at least one bore (8) through which compressed gas from the compressed gas source (4) can be conducted into the container interior space (3), wherein a compressed-gas-permeable swirl plate (9) made of a porous material is provided at the container bottom (7), wherein compressed gas can be conducted through the porous material of the compressed-gas-permeable swirl plate (9) into the container interior space (3) to aerate at least a portion of the powdered medium (30) in the container interior space (3) without conveying the powdered medium (30) out of the container interior space (3) to the dispensing unit (5), **characterized by**
a standpipe (15) which extends into the container interior space (3) and which comprises at least one intake opening (16) for drawing in powdered medium (30) stored in the container (2) so that powdered medium (30) is conveyed through the standpipe (15) to the dispensing unit (5), wherein the at least one intake opening (16) and the compressed-gas-permeable swirl plate (9) are arranged close to one another, so that the aeration of the powdered medium (30) takes place at the at least one intake opening (16) so that the drawing in of the powdered medium (30) into the standpipe (15) is favorably supported.

2. The dental device (1) according to claim 1, **characterized in that** the compressed-gas-permeable swirl plate (9) is arranged at or in a section (3A) of the container interior space (3) tapering in the direction of the container bottom (7).

3. The dental device (1) according to claim 1 or 2, **characterized in that** the compressed-gas-permeable swirl plate (9) forms an intermediate bottom limiting the container interior space (3) and separating the container interior space (3) from the container bottom (7).

4. The dental device (1) according to any one of the preceding claims, **characterized by** a compressed gas chamber (10) which is arranged beneath the compressed-gas-permeable swirl plate (9), in particular between the compressed-gas-permeable swirl plate (9) and the container bottom (7), and extends along a bottom side (9B) of the compressed-gas-permeable swirl plate (9) for supplying compressed gas to the compressed-gas-permeable swirl plate (9) over a large area.

5. The dental device (1) according to any one of the preceding claims, **characterized in that**
the compressed-gas-permeable swirl plate (9) comprises a bore (11), which preferably is disposed centrally, through which compressed gas from the compressed gas source (4) can be conducted into the container interior space (3) in order to convey the powdered medium (30) out of the container interior space (3).

6. The dental device (1) according to any one of the preceding claims, **characterized by** a first compressed gas line (12) for supplying compressed gas to the porous material of the compressed-gas-permeable swirl plate (9), preferably via the compressed gas chamber (10) extending along the bottom side (9B) of the compressed-gas-permeable swirl plate (9), so that the compressed gas conveyed through the first compressed gas line (12) passes through the porous material of the compressed-gas-permeable swirl plate (9) into the container interior space (3) of the container (2) to aerate at least a portion of the powdered medium (30) in the container interior space (3) and a second compressed gas line (13) for conducting compressed gas directly into the container interior space (3) for conveying the powdered medium (30) together with the compressed gas out of the container interior space (3), wherein the first compressed gas line (12) and the second compressed gas line (13) are designed as separate compressed gas lines independently of one another.

7. The dental device (1) according to claim 6, **characterized in that** the second compressed gas line (13) extends through the bore (11) in the compressed-gas-permeable swirl plate (9).

8. The dental device (1) according to claim 6 or 7, **characterized in that** the first compressed gas line (12) and the second compressed gas line (13) can be connected to a joint compressed gas source (4).

9. The dental device (1) according to claims 6, 7 or 8, **characterized in that** the first compressed gas line (12) and the second compressed gas line (13) are arranged in a supply block (14) that can be connected to the container (2).

10. The dental device (1) according to claim 9, **characterized in that** an extension (14A) of the supply block (14), in which a section of the second compressed gas line (13) is disposed in particular, protrudes into the bore (11) of the compressed-gas-permeable swirl plate (9).

11. The dental device (1) according to any one of claims 6 - 11, **characterized by** a standpipe (15) having a first end and a second end, wherein the first end is connected to the second compressed gas line (13) so that powdered medium (30) is drawn by the compressed gas supplied by the second compressed gas line (13) through the at least one intake opening (16) into the standpipe (15) and is conveyed with the compressed gas through the standpipe (15) in the direction of the second end.

12. The dental device (1) according to claim 11, **characterized in that** the dental device (1) in addition comprises a liquid line for conducting a liquid in the direction of the dispensing unit (5), so that the liquid can be dispensed via the dispensing unit (5) in the direction of the human or animal tissue or the tissue replacement material.

13. The dental device (1) according to any one of claims 6 - 12, **characterized by** a non-return valve (17) which is designed to prevent powdered medium (30) to flow out of the container interior space (3) into the second compressed gas line (13).

14. The dental device (1) according any one of claims 6 - 13, **characterized in that** at least the first compressed gas line (12) or the second compressed gas line (13) comprises a throttle element (18, 19) so that the volume flow of the compressed gas passing through the first compressed gas line (12) or the second compressed gas line (13) is adjustable.

15. The dental device (1) according to any one of the preceding claims, **characterized by** a dispensing unit (5), in particular a handle (5A), having a nozzle arrangement (5B) which is designed for dispensing powdered medium (30) in the direction of human or animal tissue or a tissue replacement material.

## Revendications

1. Dispositif de médecine dentaire (1) pour la distribution d'un milieu pulvérulent (30) sur du tissu humain ou animal ou sur une matière tissulaire de remplacement, avec un récipient (2) pour la conservation du milieu pulvérulent (30) dans un espace intérieur de récipient (3), dans lequel le récipient (2) peut être raccordé à une source de gaz comprimé (4) et du gaz comprimé de la source de gaz comprimé (4) peut être conduit dans l'espace intérieur de récipient (3) pour transporter, avec le gaz comprimé, le milieu pulvérulent (30) hors de l'espace intérieur du récipient (3), et dans lequel le récipient (2) peut être relié à une unité de distribution (5) de sorte que le milieu pulvérulent (30) est transporté avec le gaz comprimé vers l'unité de distribution (5) et peut être distribué par l'unité de distribution (5) en direction du tissu humain ou animal ou de la matière tissulaire de remplacement, dans lequel le récipient (2) présente: une paroi de récipient (6) qui définit au moins une partie de l'espace intérieur de récipient (3), et un fond de récipient (7), relié à la paroi de récipient (6), avec au moins un alésage (8) à travers lequel du gaz comprimé de la source de gaz comprimé (4) peut être conduit dans l'espace intérieur de récipient (3), dans lequel on prévoit, sur le fond de récipient (7), une plaque de tourbillonnement (9) perméable au gaz en un matériau poreux, dans lequel, à travers le matériau poreux de la plaque de tourbillonnement (9) perméable au gaz, du gaz comprimé peut être conduit dans l'espace intérieur de récipient (3) pour ameublir au moins une partie du milieu pulvérulent (30) dans l'espace intérieur de récipient (3) sans transporter le milieu pulvérulent (30) depuis l'espace intérieur de récipient (3) vers l'unité de distribution (5), **caractérisé par**
un tube montant (15) qui s'étend dans l'espace intérieur de récipient (3) et qui présente au moins une ouverture d'aspiration (16) pour aspirer du milieu pulvérulent (30) conservé dans le récipient (2) pour transporter le milieu pulvérulent (30) à travers le tube montant (15) vers l'unité de distribution (5), dans lequel l'au moins une ouverture d'aspiration (16) et la plaque de tourbillonnement (9) perméable au gaz sont disposées à proximité l'une de l'autre de sorte que l'ameublissement du milieu pulvérulent (30) s'effectue au niveau de l'au moins une ouverture d'aspiration (16) moyennant quoi l'aspiration du milieu pulvérulent (30) dans le tuyau montant (15) est favorisée.

2. Dispositif de médecine dentaire (1) selon la revendication 1, **caractérisé en ce que** la plaque de tourbillonnement (9) perméable au gaz est disposée au niveau de, ou dans une partie (3A) de l'espace intérieur de récipient (3) s'amenuisant en direction du fond du récipient (7).

3. Dispositif de médecine dentaire (1) selon la revendication 1 ou 2, **caractérisé en ce que**
la plaque de tourbillonnement (9) perméable au gaz forme un fond intermédiaire délimitant l'espace intérieur de récipient (3), lequel sépare l'espace intérieur de récipient (3) du fond de récipient (7).

4. Dispositif de médecine dentaire (1) selon l'une des revendications précédentes, **caractérisé par**
une chambre de gaz comprimé (10), disposée au-dessous de la plaque de tourbillonnement (9) perméable au gaz, en particulier entre la plaque de tourbillonnement (9) perméable au gaz et le fond de récipient (7), s'étendant le long d'un dessous (9B) de la plaque de tourbillonnement (9) perméable au gaz pour l'alimentation en nappe de la plaque de tourbillonnement (9) perméable au gaz avec du gaz comprimé.

5. Dispositif de médecine dentaire (1) selon l'une des revendications précédentes, **caractérisé en ce que**
la plaque de tourbillonnement (9) perméable au gaz présente un alésage (11), de préférence disposé centralement, à travers lequel du gaz comprimé de la source de gaz comprimé (4) peut être conduit dans l'espace intérieur de récipient (3) pour transporter le milieu pulvérulent (30) hors de l'espace intérieur de récipient (3).

6. Dispositif de médecine dentaire (1) selon l'une des revendications précédentes, **caractérisé par**
une première conduite de gaz comprimé (12) pour l'alimentation du matériau poreux de la plaque de tourbillonnement (9) perméable au gaz avec du gaz comprimé, de préférence via la chambre de gaz comprimé (10) s'étendant le long du dessous (9B) de la plaque de tourbillonnement (9) perméable au gaz, de sorte que le gaz comprimé transporté à travers la première conduite de gaz comprimé (12) gagne l'espace intérieur de récipient (3) du récipient (2) à travers le matériau poreux de la plaque de tourbillonnement (9) perméable au gaz pour ameublir au moins une partie du milieu pulvérulent (30) dans l'espace intérieur de récipient (3), et une deuxième conduite de gaz comprimé (13) pour directement conduire du gaz comprimé dans l'espace intérieur de récipient (3) pour transporter, avec le gaz comprimé, le milieu pulvérulent (30) hors de l'espace intérieur de récipient (3), dans lequel la première conduite de gaz comprimé (12) et la deuxième conduite de gaz comprimé (13) sont réalisées en tant que conduites de gaz comprimé séparées l'une de l'autre.

7. Dispositif de médecine dentaire (1) selon la revendication 6, **caractérisé en ce que** la deuxième conduite de gaz comprimé (13) s'étend à travers l'alésage (11) de la plaque de tourbillonnement (9) perméable au gaz.

8. Dispositif de médecine dentaire (1) selon la revendication 6 ou 7, **caractérisé en ce que**
la première conduite de gaz comprimé (12) et la deuxième conduite de gaz comprimé (13) peuvent être raccordées à une source de gaz comprimé (4) commune.

9. Dispositif de médecine dentaire (1) selon la revendication 6, 7 ou 8, **caractérisé en ce que**
la première conduite de gaz comprimé (12) et la deuxième conduite de gaz comprimé (13) sont disposées dans un bloc d'alimentation (14) pouvant être raccordé au récipient (2).

10. Dispositif de médecine dentaire (1) selon la revendication 9, **caractérisé en ce qu'**un prolongement (14A) du bloc d'alimentation (14), dans lequel est en particulier disposée une partie de la deuxième conduite de gaz comprimé (13), fait saillie dans l'alésage (11) de la plaque de tourbillonnement (9) perméable au gaz.

11. Dispositif de médecine dentaire (1) selon l'une des revendications 6 - 11, **caractérisé par**
un tube montant (15) avec une première extrémité et une deuxième extrémité, dans lequel la première extrémité est en jonction avec la deuxième conduite de gaz comprimé (13) de sorte que du milieu pulvérulent (30) est aspiré par le gaz comprimé fourni par la deuxième conduite de gaz comprimé (13) à travers l'au moins une ouverture d'aspiration (16) dans le tube montant (15) et est transporté avec le gaz comprimé à travers le tube montant (15) en direction de la deuxième extrémité.

12. Dispositif de médecine dentaire (1) selon la revendication 11, **caractérisé en ce que** le dispositif de médecine dentaire (1) présente en outre une conduite de liquide pour conduire un liquide en direction de l'unité de distribution (5) de sorte que le liquide peut être distribué via l'unité de distribution (5) en direction du tissu humain ou animal ou de la matière tissulaire de remplacement.

13. Dispositif de médecine dentaire (1) selon l'une des revendications 6 - 12, **caractérisé par**
un clapet anti-retour (17) qui est réalisé pour empêcher la pénétration d'un milieu pulvérulent (30) depuis l'espace intérieur de récipient (3) dans la deuxième conduite de gaz comprimé (13).

14. Dispositif de médecine dentaire (1) selon l'une des revendications 6 - 13, **caractérisé en ce**
**qu'**au moins la première conduite de gaz comprimé (12) ou la deuxième conduite de gaz comprimé (13) présente un élément étrangleur (18, 19) de sorte que le débit de gaz comprimé circulant à travers la première conduite de gaz comprimé (12) ou la deuxième conduite de gaz comprimé (13) peut être réglé.

15. Dispositif de médecine dentaire (1) selon l'une des revendications précédentes, **caractérisé par**
une unité de distribution (5), en particulier une poignée (5A), avec un dispositif de buse (5B) réalisé pour la distribution du milieu pulvérulent (30) en direction du tissu humain ou animal ou de la matière tissulaire de remplacement.
